# FASCICULE DE BREVET EUROPEEN

(11) **EP 4 019 015 B1**
(45) Date de publication et mention de la délivrance du brevet: **11.12.2024**
(21) Numéro de dépôt: 21215012.2
(22) Date de dépôt: 16.12.2021
(51) Int. Cl.: A61K 31/164, A61K 31/198, A61K 31/401, A61K 31/4172, A61K 8/44, A61K 8/60, A61P 17/00

(54) **COMPOSITION COSMETIQUE APTE A RENFORCER LES JONCTIONS SERREES EPIDERMIQUES POUR LA PREVENTION ET/OU LE TRAITEMENT DE LA DERMATITE ATOPIQUE**
KOSMETISCHE ZUSAMMENSETZUNG ZUR STÄRKUNG DER EPIDERMALEN TIGHT JUNCTIONS ZUR VORBEUGUNG UND/ODER BEHANDLUNG VON ATOPISCHER DERMATITIS
COSMETIC COMPOSITION CAPABLE OF REINFORCING EPIDERMAL TIGHT JUNCTIONS FOR THE PREVENTION AND/OR TREATMENT OF ATOPIC DERMATITIS

(30) Priorité: 24.12.2020 FR 2014142
(43) Date de publication de la demande: 29.06.2022
(73) Titulaire: NAOS Institute of Life Science, 13290 Aix-en-Provence (FR); Thorel, Jean-Noël, 75014 Paris (FR)
(72) Inventeur: THOREL, Jean-Noël, 75014 PARIS (FR)
(74) Mandataire: Cabinet Laurent & Charras

(56) Documents cités:
- JP-A- 2000 264 825
- JP-A- 2000 264 829
- JP-A- 2001 026 530
- US-A1- 2012 172 326
- MAN MAO-QIANG ET AL: "Optimization of Physiological Lipid Mixtures for Barrier Repair.", JOURNAL OF INVESTIGATIVE DERMATOLOGY, vol. 106, no. 5, 1 May 1996 (1996-05-01), pages 1096 - 1101, XP055031349, ISSN: 0022-202X, DOI: 10.1111/1523-1747.ep12340135

## Description

### Domaine technique de l'invention

L'invention se rapporte au domaine des soins dermatologiques, et plus particulièrement du traitement des affections inflammatoires cutanées. Plus précisément, l'invention concerne une composition, avantageusement cosmétique, pour la prévention et/ou le traitement de la dermatite atopique.

### Arrière-plan technologique

La dermatite atopique, ou atopie cutanée, est une dermatose inflammatoire chronique prurigineuse, c'est-à-dire, une affection inflammatoire de la peau qui se manifeste par un prurit intense et chronique et par des lésions eczémateuses récurrentes évoluant par poussées (Mack and Kim, 2018; Weidinger *et al.,* 2018). Cette pathologie est liée à des anomalies de la fonction de la barrière cutanée ou encore à l'apparition éventuelle d'autres symptômes tels que rhinite allergique, asthme, hypersensibilité à des allergènes de l'environnement qui sont normalement tolérés chez des sujets sains. JP2000264829 divulgue une composition comprenant au moins un copolymère d'acide acrylique, une glucosylcéramide; un ingrédient huileux, et au moins un acide aminé, en particulier l'arginine utile pour traiter la dermatite atopique.

L'altération de la fonction barrière de l'épiderme joue un rôle clé dans l'initiation de la dermatite atopique (Kuo *et al.,* 2013). En effet, ce défaut de fonction barrière de l'épiderme induit un cercle vicieux de démangeaisons-grattage (Mochizuki *et al.,* 2019), ce qui augmente la perméabilité aux allergènes, bactéries et virus (Cipriani et al., 2014; Halling-Overgaard et al., 2017), qui agissent à leur tour en tant que médiateurs du processus d'inflammation accru.

Les jonctions serrées (ou *zonula occludens*) sont des jonctions intercellulaires cruciales pour l'adhésion épithéliale (ou adhésion intercellulaire) et la fonction barrière. Il s'agit de complexes transmembranaires dynamiques qui s'ouvrent et se ferment. Ces jonctions serrées sont composées de protéines de structures, de protéines associées au cytosquelette et de protéines participant aux mécanismes de signalisation et de polarité cellulaire (Niessen, 2007).

En particulier, les jonctions serrées sont constituées de protéines transmembranaires comme les claudines, les occludines, les molécules d'adhésion jonctionnelles (JAMs), et les tricellulines qui sont des protéines de structure similaires aux occludines ; et les protéines cytosoliques comme les protéines *zonula occludens* (ZO), les cingulines, la protéine *multiple-PDZ domain protein* 1 (MUPP1) ou encore la protéine *Membrane-Associated Guanylate kinase with Inverted orientation* (MAGI).

Les jonctions serrées se retrouvent en particulier dans la couche granuleuse (ou *stratum granulosum*) de l'épiderme (Brandner *et al.,* 2002).

La principale fonction des jonctions serrées dans la peau est l'établissement d'une deuxième barrière cutanée qui intervient instantanément lors de la rupture de la couche cornée de la peau (ou *stratum corneum*)*.*

Plusieurs études scientifiques montrent que l'expression de plusieurs protéines composant les jonctions serrées est modifiée dans les peaux de patients atteints de dermatite atopique, notamment l'expression de la claudine-1 est diminuée de façon significative dans les zones lésionnelles de patients atteints de dermatite atopique (Batista *et al.,* 2015; Gruber *et al.,* 2015; Yuki *et al.,* 2011).

Il ressort de ce qui précède que la restauration de l'expression et de la fonctionnalité des jonctions serrées offre une nouvelle voie pour renforcer ou rétablir la fonction barrière de la peau, et de ce fait, prévenir et/ou traiter la dermatite atopique.

A titre d'exemple, les documents EP2 691 090 et EP 2 704 704 décrivent respectivement l'utilisation de principe actifs cosmétiques (polyphénol végétal, flavonoïdes ou cis-acide urocanique) et des bactéries probiotiques pour renforcer les jonctions serrées dans le but de traiter la dermatite atopique.

Toutefois, les solutions proposées par ces documents correspondent à des compositions non définies d'extraits de plante et lysats bactériens qui peuvent donner lieu à des réactions allergiques ou inflammatoires suite à l'application sur peau humaine et dont la composition peut varier de façon significative de lot à lot.

Il subsiste donc un besoin évident de mettre au point une composition comprenant des composés actifs dermatologiques purs qui contribuent à rétablir la fonctionnalité des jonctions serrées dans les peaux pre-atopiques et atopiques, qui soient également hautement titrés en molécules actives permettant de prévenir et/ou traiter la dermatite atopique, notamment à long terme, et qui soient sûrs pour l'utilisation topique humaine, c'est-à-dire, parfaitement compatibles avec la peau et idéalement, biomimétiques, c'est-à-dire identiques à des constituants de la peau humaine.

### Description de l'invention

Le Demandeur a constaté que, de manière surprenante, plusieurs principes actifs dermatologiques, qui sont parfaitement sûrs et adaptés à l'utilisation cosmétique humaine, permettent de surmonter les inconvénients et de répondre aux besoins précédemment décrits.

L'invention vise, de manière générale, à répondre à un besoin, notamment cosmétique, réel en hydratant et apaisant la peau, en réduisant les sensations d'inconfort et, *in fine,* en améliorant l'aspect de la peau.

En particulier, l'invention vise à traiter la dermatite atopique afin d'améliorer l'aspect de la peau et réduire les imperfections.

Plus précisément, le Demandeur a constaté que les composés, ou principes actifs, compris dans la composition selon l'invention agissent de façon synergique pour induire l'expression de la claudine-1, une protéine structurelle clé des jonctions serrées. Ces composés, qui sont parfaitement biomimétiques, se prêtent à leur utilisation pour la formulation de produits dermo-cosmétiques adaptés à la prévention et/ou le traitement de la dermatite atopique.

Au sens de l'invention, par « principe actif » on désigne une substance ou un composé qui possède des propriétés biologiques et/ou thérapeutiques qui sous-tendent un effet physiologique.

Le principe actif est à différencier du au moins un excipient, présent dans la composition selon l'invention.

Par « excipient » on désigne une autre substance que le principe actif qui confère à la composition des propriétés, notamment, de consistance, galénique et/ou de vectorisation du principe actif.

Au sens de l'invention, par « composé biomimétique » on désigne un composé qui existe naturellement sur/dans une peau saine et ses annexes ou qui, après hydrolyse ou oxydo/réduction, se transforme en composés qui eux aussi, existent naturellement sur/dans une peau saine et ses annexes. Selon l'invention, ce composé est considéré à l'état isolé, sous une forme identique à sa forme naturelle, ou modifiée par rapport à sa forme naturelle, mais restant cytocompatible avec la peau quel que soit son mode d'obtention ou de production.

Ainsi et selon un premier aspect, l'invention concerne une composition comprenant,
- au moins un acide aminé basique, avantageusement sélectionné dans le groupe constitué de l'arginine, la lysine, l'histidine, l'ornithine et l'hydroxyproline ; et
- au moins un glycosylcéramide.

Selon un mode de réalisation particulier, la composition selon l'invention est une composition cosmétique ou dermo-pharmaceutique, avantageusement cosmétique.

Les acides aminés sont des actifs totalement biomimétiques car ils se retrouvent dans tous les organismes vivants, soit en qualité de constituants de protéines, soit sous forme libre consécutivement à un processus de dégradation d'un organisme vivant.

Au sens de l'invention, par « acide aminé basique » on désigne toutes les formes de l'acide aminé, dont tout sel.

Les glycosylcéramides sont des glycolipides constitués d'une céramide liée par une liaison bêta-osidique (β-osidique) à un seul ose neutre (monoglycosylcéramide) ou à plusieurs oses neutres (polyglycosylcéramide), une céramide étant l'amide d'une sphingosine et d'un acide gras. Avantageusement, l'ose neutre est un hexose ou un désoxyhexose sélectionné dans le groupe constitué par l'allose, l'altrose, le glucose, le mannose, le gulose, l'idose, le galactose, le fucose, fructose. L'acide gras est avantageusement à longue chaine et contient en général de 16 à 18 atomes de carbone, la chaine étant éventuellement hydroxylée. De préférence, les céramides sont choisies dans le groupe comprenant céramide 1, céramide 2, céramide 3, céramide 4, céramide 5, céramide 6, céramide 7.

Les glycosylcéramides se retrouvent dans tous les organismes, notamment l'Homme où ils sont particulièrement abondants dans les tissus nerveux et cérébraux en qualité de constituants de la gaine de myéline. De ce fait, les glycosylcéramides sont considérés comme des composés biomimétiques.

Selon un mode de réalisation particulier, l'acide aminé basique compris dans la composition selon l'invention est l'arginine ou un de ses sels.

Les différentes formes de l'arginine sont, en général, la D-arginine, la D,L-arginine et la L-arginine. Parmi les sels d'acide organique ou minéral d'arginine, on peut citer par exemple les chlorhydrates, les glutamates, butyrates, et glycolates.

Avantageusement, la composition selon l'invention contient de la L-arginine.

Selon un mode de réalisation particulier, l'arginine utilisée dans la composition selon l'invention est sous forme purifiée, de préférence de pureté au moins égale à 60%, de préférence au moins égale à 70%, avantageusement au moins égale à 80%, encore plus avantageusement au moins égale à 90%, voire au moins égale à 95% voire 98%. L'arginine contenue dans la composition selon l'invention peut être d'origine végétale, chimique, ou bien obtenus par biotechnologie. Ainsi la matière première cosmétique L-Arginine commercialisée par la société Kyowa Hakko Kogyo Co., Ltd et correspondant à la désignation INCI arginine peut être utilisée dans la composition selon l'invention.

Selon un mode de réalisation particulier, la composition selon l'invention comprend au moins un glycosylcéramide se présentant sous la forme d'un monoglycosylcéramide et/ou d'un polyglycosylcéramide, avantageusement d'un monoglycosylcéramide et/ou tétraglycosylcéramide.

Selon un mode de réalisation préféré, la composition selon l'invention comprend des glycosylcéramides sous forme monoglycosylcéramide et tétraglycosylcéramide.

Selon un mode de réalisation particulier, la composition comprend un monoglycosylcéramide correspondant à un acide glucuronique (ou acide gluconique) lié de façon covalente par une liaison β-osidique à un céramide.

Selon un mode de réalisation particulier, le polyglycosylcéramide selon l'invention correspond à un oligosaccharide lié de façon covalente par une liaison β-osidique à un céramide.

Avantageusement, le polyglycosylcéramide selon l'invention est un tétraglycosylcéramide dont l'oligosaccharide correspond à l'enchaînement des oses suivant : acide glucuronique-glucosamine-galactose-mannose (ou GlcA-GlcN-Gal-Man).

Selon un mode de réalisation particulier, le ou les glycosylcéramides selon l'invention sont sous forme purifiée, de pureté au moins égale à 60%, de préférence au moins égale à 70%, avantageusement au moins égale à 80%, encore plus avantageusement au moins égale à 90%, voire au moins égale à 95%.

Le ou les glycosylcéramides compris dans la composition selon l'invention peuvent être d'origine végétale, chimique, ou bien obtenus par biotechnologie.

Avantageusement, la composition selon l'invention comprend au moins un glycosylcéramide d'origine biotechnologique.

Avantageusement, tous les glycosylcéramides compris dans la composition selon l'invention sont d'origine biotechnologique.

Selon un mode de réalisation particulier, les glycosylcéramides de l'invention sont d'origine biotechnologique, notamment issus de l'extraction des membranes d'espèces bactériennes les synthétisant, comme par exemple *Pseudomonas spp.* Avantageusement, les glucosylcéramides sont obtenus à partir de l'espèce bactérienne *Pseudomonas paucimobilis.* Ainsi, les matières premières cosmétiques Glycosphingolipid et Biosphingo, commercialisées respectivement par les sociétés THREE B CO., INC ET KIKKOMAN BIOCHEMIFA COMPANY, et correspondant à la désignation INCI glycosphingolipids, peuvent être utilisés dans la composition selon l'invention.

Selon un mode de réalisation particulier, le au moins un acide aminé basique, avantageusement l'arginine ou un de ses sels, représente entre 0,001 % et 2% en poids total de la composition, avantageusement entre 0,01 % et 0,5%.

Selon un autre mode de réalisation particulier, au moins un glycosylcéramide, avantageusement des glycosylcéramides sous forme monoglycosylcéramide et tétraglycosylcéramide, représente entre 0,0001% et 1% en poids total de la composition, avantageusement entre 0,001% et 0,1%.

Dans un mode de réalisation particulier, la composition selon l'invention comprend, en outre au moins un saponoside triterpénique ou l'un de ses dérivés choisi dans le groupe constitué de l'acide glycyrrhétinique, la glycyrrhizine, le stéaryl glycyrrhétinate et le dipotassium diglycyrrizinate.

Ces composés, issus de la réglisse, ont des propriétés anti-inflammatoires et sont couramment employés dans le traitement topique de la dermatite atopique pour combattre le prurit associé avec cette condition de la peau. A titre d'exemple, les matières premières cosmétique 18β-glycyrrhetic acid, dipotassium glycyrrhizinate et glycyrrhizic acid et correspondant respectivement aux désignations INCI glycyrrhetinic acid, dipotassium glycyrrhizate et glycyrrhizic acid et au stearyl glycyrrhetinate (tous désignés par leur dénomination INCI et commercialement accessibles auprès des producteurs listés par exemple dans le International Buyer's Guide de l'INCI) peuvent être utilisées dans les compositions selon l'invention.

Avantageusement, la composition selon l'invention comprend, en outre de l'acide aminé basique et du au moins un glycosylcéramide, une association de l'acide glycyrrhétinique et le dipotassium diglycyrrizinate en qualité d'agents anti-inflammatoires.

Selon un mode de réalisation particulier, au moins un saponoside triterpénique ou l'un de ses dérivés représente entre 0,0001% et 10% en poids total de la composition, avantageusement entre 0,01% et 5%, de préférence entre 0,05% et 1 %.

Selon un mode de réalisation, la composition selon l'invention comprend, en outre, au moins un tensioactif non-ionique sélectionné parmi les esters de saccharose et/ou les esters de sorbitan.

Selon un mode de réalisation particulier, le au moins un tensioactif non-ionique selon l'invention représente 0,1% et 5% en poids total de la composition, avantageusement entre 1% et 3%.

Les esters de saccharose et/ou esters de sorbitan adaptés selon l'invention sont décrits en détail dans le document WO2014/023895 incorporé entièrement par référence. Ces agents, seuls ou en association, permettent de réduire l'adhésion et/ou la prolifération du staphylocoque doré sur la peau et/ou sur la muqueuse nasale, améliorant ultérieurement le cadre clinique général de la dermatite atopique.

Selon un mode de réalisation préféré, le tensioactif non-ionique de l'invention correspondant à un ester de sorbitan est le polysorbate 20 (INCI) ou le polysorbate 80 (INCI), avantageusement le polysorbate 20.

Selon un autre mode de réalisation préféré, le tensioactif non-ionique de l'invention correspondant à un ester de saccharose est un sucrostéarate ayant une valeur de Balance Hydrophile Lipophile (HLB) d'au moins 16, ce qui correspond à une proportion relative en monoesters de sucrose et d'acide stéarique comprise entre 75% et 80% en poids du sucrose stéarate (INCI).

Selon un mode de réalisation particulier, le tensioactif non-ionique de l'invention représente entre 0,1% et 5% en poids de la composition, avantageusement entre 1% et 3%.

Selon un mode de réalisation particulier, la composition selon l'invention comprend, en outre, au moins un lipide apte à restaurer la barrière cutanée, avantageusement sélectionné dans le groupe constitué de :
- au moins un lipide exogène de la peau, avantageusement une huile végétale ;
- un mélange de constituants naturellement présents dans la peau comprenant des céramides 1, 3, 6 ; du cholestérol ; des acides gras libres et de la phytosphingosine ; et
- le squalane.

Dans un mode de réalisation particulier, les lipides exogènes de la peau correspondent à l'huile de tournesol (INCI : *Helianthus annuus* seed oil), l'huile de colza (INCI : canola oil) et/ou l'huile de jojoba (INCI : *Simmondsia chinensis* (jojoba) seed oil). Ces trois huiles végétales ont des propriétés émollientes et anti-inflammatoires connues.

Dans un mode de réalisation particulier, le mélange de constituants naturellement présents dans la peau se présente sous la forme d'une composition contenant les lipides répondant aux noms INCI suivants : Sodium Lauroyl Lactylate, Ceramide NP, Ceramide AP, Phytosphingosine, Cholesterol, Ceramide EOP.

Dans un mode de réalisation particulier, le lipide apte à restaurer la barrière cutanée est le squalane, un lipide qui se retrouve également dans la composition de la peau. Le squalane au sens de l'invention est avantageusement d'origine végétale.

De préférence, les lipides aptes à restaurer la barrière cutanée représentent entre 0,01% et 25% en poids de la composition, avantageusement entre 1 % et 15%.

Selon un mode de réalisation particulier, la composition de l'invention comprend, en outre, au moins un composé polyhydroxylé additionnel choisi dans le groupe comprenant le rhamnose, le xylitol et le mannitol. Ces composés polyhydroxylés contribuent à réduire l'adhésion des bactéries pathogènes, comme *S*. *aureus,* sur la peau et les muqueuses chez l'Homme, dont la muqueuse nasale. Le mannitol, quant à lui, possède une activité antiradicalaire.

Dans un mode de réalisation particulier de l'invention, la composition comprend un mélange des trois composés polyhydroxylés additionnels cités ci-dessus. Avantageusement, le rhamnose représente entre 0,01% et 1% en poids total de la composition, le xylitol représente entre 0,05% et 2% en poids total de la composition et le mannitol représente entre 0,005% et 1% en poids total de la composition.

De manière avantageuse, la composition de l'invention comprend, en outre, de la vitamine PP, aussi appelée vitamine B3, nicotinamide ou niacinamide, ou l'un de ses dérivés, composés connus pour stimuler la synthèse des lipides de la couche cornée, telles que les céramides, les acides gras libres et le cholestérol. La vitamine PP agit en stimulant l'activité de la sérine palmitoyl transférase, une enzyme clef de la synthèse de sphingosine, molécule précurseur des céramides.

Selon un mode de réalisation particulier, la niacinamide et/ou l'un de ses dérivés représentent entre 0,001% et 10 % en poids total de la composition, avantageusement entre 0,01% et 5 %, de préférence entre 0,1 % et 2%.

Selon un mode de réalisation particulier, la composition de l'invention comprend des substances prébiotiques, notamment des oligosaccharides comme des fructooligosaccharides ou FOS (INCI), des dextrines (INCI) à chaines courte, des arabinogalactanes (INCI), de l'inuline (INCI) ou du lactulose (INCI). Dans un mode de réalisation préféré, la composition selon l'invention contient des FOS, c'est-à-dire des oligomères de fructose et saccharose ayant un degré de polymérisation compris entre 2 et 10. Les FOS contribuent à l'induction de β-défensines et sont également métabolisés par la flore saprophyte. Les FOS ont des propriétés antimicrobiennes et permettent de réduire l'utilisation de conservateurs artificiels comme décrit dans le document FR 2 831 059.

Avantageusement, les FOS représentent entre 0,001% et 20 % en poids total de la composition, avantageusement entre 0,01% et 10 %, de préférence entre 0,1 % et 5 %.

Selon un mode de réalisation particulier, la composition selon l'invention comprend, en outre, au moins un inducteur des peptides antimicrobiens, c'est-à-dire un ingrédient actif capable d'induire la synthèse et/ou sécrétion des peptides antimicrobiens chez l'Homme (notamment, des β-défensines) ou par le microbiote. Ces propriétés ont été mises en évidence notamment dans des extraits végétaux, tels que l'extrait de *Peumus boldus* (boldo) ou l'extrait de *Filipendula ulmaria* (reine-des-prés).

Avantageusement, l'inducteur de peptides antimicrobiens est l'extrait de boldo, l'extrait de la reines-des-prés ou leur mélange.

Selon un mode de réalisation particulier, l'inducteur de peptides antimicrobiens représente entre 0,001% et 5 % en poids total de la composition, de préférence entre 0,01% et 1 %.

Selon un mode de réalisation particulier, la composition selon l'invention se présente sous une forme adaptée pour être cosmétiquement et/ou dermatologiquement acceptable, c'est-à-dire compatible avec la peau, les muqueuses, les cheveux, les ongles et/ou le cuir chevelu.

Selon un mode de réalisation préféré, la composition selon l'invention se présente sous une forme adaptée à une administration par voie topique.

Dans un autre mode de réalisation, la composition avantageusement cosmétique selon l'invention comprend en outre, un système bioactif associant d'une part, une forme stable en solution aqueuse d'un nucléotide choisi parmi l'ATP (adenosine triphosphate) le Gp4G (diguanosine tetraphosphate) et l'Ap4A (diadénosine tetraphosphate) ; et d'autre part, au moins un peptide biomimétique comprenant au plus six acides aminés, avantageusement différents de celui de l'invention, mimant un polypeptide cutané ou une protéine cutanée, ou une biomolécule agoniste ou antagoniste dudit peptide ou de ladite protéine. En pratique, l'association de ces principes actifs permet de catalyser l'activité métabolique des cellules de la peau tout en obtenant un effet dermocosmétique ou thérapeutique grâce à l'utilisation des peptides biomimétiques. Ces derniers peuvent être sélectionnés afin d'obtenir l'effet souhaité, par exemple, un effet d'inhibition des irritations d'origine neurogène, une activité dépigmentante, un effet inhibant toute intolérance ou sensibilisation etc.

En pratique, dans le système bioactif selon l'invention, le nucléotide représente au plus 10% en poids total de la composition, de préférence entre 0,001% et 5% ; et le peptide biomimétique représente entre 0,001% à 1 % en poids total de la composition.

Selon un autre mode de réalisation, la composition selon l'invention peut comprendre, en outre, un extrait de la bactérie *Arthrobacter agilis,* notamment un extrait riche en caroténoïdes INCI : Micrococcus lysate). Avantageusement, la composition selon l'invention comprend un tel extrait sec qui représente entre 0,00001 % et 0,1 % en poids total de la composition, de préférence entre 0,0001% et 0,001%.

Selon un autre mode de réalisation, la composition selon l'invention comprend, en outre, d'autres composants pouvant contribuer à la protection interne par une action qui peut consister en une protection de l'ADN, une diminution de l'immunosuppression induite par les radiations UV, une action antiradicalaire ou un effet combiné de ces actions.

L'action protectrice d'une préparation selon l'invention contre le stress oxydatif ou à l'encontre de l'effet des radicaux libres peut être encore améliorée si celle-ci comprend, en outre, un ou plusieurs antioxydants, aisément sélectionnées par l'Homme du métier parmi les matières premières utilisés dans le domaine cosmétique.

Dans un mode de réalisation particulier de l'invention, la composition selon l'invention contient également des principes actifs aptes à stimuler la prolifération de cellules cutanées, avantageusement des fibroblastes et/ou de kératinocytes, de préférence des fibroblastes humains ou animaux. En particulier et selon ce mode de réalisation, la composition selon l'invention comprend, en outre :
- de l'acide α-lipoïque ou l'un de ses sels ;
- un dérivé de vitamine C choisi parmi l'éthyle acide ascorbique et l'ascorbate de sodium ou leur mélange ; et
- de l'acide hyaluronique, dont le poids moléculaire (Mw) est avantageusement compris entre 0,5 et 15 kDa, de préférence 0,5 et 10 kDa.

Avantageusement, l'acide α-lipoïque ou l'un de ses sels représente moins de 0,1% en poids total de la composition, avantageusement moins de 0,01%, de préférence entre 0,001% et 0,0005%.

Avantageusement, le dérivé de vitamine C choisi parmi l'éthyle acide ascorbique et l'ascorbate de sodium ou leur mélange, avantageusement l'éthyle acide ascorbique, de préférence le 3-O éthyl éther d'acide ascorbique, représente entre 0,001% et 10% en poids total de la composition, avantageusement entre 0,01% et 5%, de préférence entre 0,1% et 1%.

Avantageusement, l'acide hyaluronique, dont le poids moléculaire (Mw) est avantageusement compris entre 0,5 et 15 kDa, de préférence 0,5 et 10 kDa, représente entre 0,001% et 10% en poids total de la composition, avantageusement entre 0,01% et 5%, de préférence entre 0,1 % et 1 %.

Selon un autre mode de réalisation de l'invention, la composition comprend, en outre, au moins un, voire tous les constituants suivants exerçant une activité biologique in *vivo* sur les cellules de la peau, des lèvres, des cheveux et/ou des muqueuses soumises à un rayonnement UV-A et/ou UV-B, respectivement :
- un agent antiradicalaire préservant les structures cellulaires, tel que par exemple la vitamine E et/ou ses dérivés liposolubles ou hydrosolubles, en particulier le tocotriénol et/ou le tocophérol, représentant avantageusement entre 0,001% et 10% en poids total de la composition, encore plus avantageusement entre 0,02% et 2%, de préférence 0,04% ;
- un agent limitant l'immunosuppression, tel que par exemple la vitamine PP, représentant avantageusement entre 0,001% et 1 % en poids total de la composition, de préférence entre 0,01% et 0,3%;
- un agent protecteur de la protéine p53, tel que par exemple l'épigallocatéchine gallate (EGCG), représentant avantageusement entre 0,001% et 0,1% en poids total de la composition, de préférence entre 0,005% et 0,05%.

La composition selon l'invention peut également comprendre, en outre, des extraits peptidiques de soja et/ou de blé.

En pratique, les extraits peptidiques provenant de graines de soja ou de blé sont issu d'une hydrolyse enzymatique desdites graines par l'intermédiaire de peptidases qui permet de récupérer des peptides d'une taille moyenne de 700 Da. Préférentiellement, l'extrait peptidique de soja est l'extrait identifié sous le numéro CAS 68607-88-5 et/ou l'extrait peptidique de blé est l'extrait identifié sous le numéro CAS 70084-87-6. Les extraits de blé et soja peuvent correspondre aux désignations INCI Hydrolyzed wheat protein et Hydrolyzed soy protein, respectivement.

Dans un mode de réalisation particulier, les extraits peptidiques de soja et de blé sont utilisés ensemble, par exemple dans un rapport pondéral respectivement compris entre 80/20 et 20/80, avantageusement compris entre 70/30 et 30/70, de préférence égal à 60/40.

Dans un mode de réalisation avantageux, les extraits peptidiques de soja et/ou de blé sont exempts de tripeptides synthétiques GHK (glycyl-histidyl-lysine ; INCI : Tripeptide-1). En pratique, les extraits peptidiques de soja et/ou blé représentent entre 0,01% et 20% en poids total de la composition, avantageusement entre 0,1 % et 10%, de préférence entre 0,2% et 0,7%.

Selon un mode de réalisation alternatif et de manière avantageuse, la composition selon l'invention comprend, en outre, au moins un filtre UV. La filtration de la lumière UV est connue pour améliorer l'état générale de la peau dans les individus atteints par la dermatite atopique.

Au sens de l'invention, le terme « filtre UV» englobe les composés organiques ou minéraux capables de filtrer les UV-A, les UV-B et/ou les UV-C.

Selon l'invention, il peut aussi bien s'agir de filtres minéraux que de filtres chimiques ou organiques.

Les compositions selon l'invention peuvent contenir un ou plusieurs filtres UV à large spectre, c'est-à-dire des composés ou des mélanges qui absorbent les UV-A, les UV-B, les UV-C et éventuellement la lumière visible.

Parmi les filtres organiques à large spectre, les filtres correspondant aux désignations INCI suivantes peuvent être utilisés dans le cadre de l'invention : tris biphenyl triazine, bis ethylhexyloxyphenol methoxyphenyl triazine, methylene bis-benzotriazolyl tetramethylbutylphenol.. Un autre exemple de filtre à large spectre adapté à la composition selon l'invention répond à la désignation INCI diethylhexyl butamido triazone.

Ainsi et dans un mode de réalisation particulier, la composition selon l'invention comprend au moins un filtre choisi dans le groupe suivant de composés identifiés par leur désignation INCI : tris biphenyl triazine, bis ethylhexyloxyphenol methoxyphenyl triazine, et methylene bis-benzotriazolyl tetramethylbutylphenol, diethylhexyl butamido triazone, ou leurs mélanges.

Avantageusement, la composition comprend le filtre bis-ethylhexyloxyphenol methoxyphenyl triazine.

Selon un autre mode de réalisation, à la place ou en plus du ou des filtres à large spectre, la composition contient au moins un filtre UV-A et/ou UV-B, organique et/ou minéral, qui peut se présenter en phase aqueuse (lipophile) et/ou huileuse (liposoluble).

Ainsi et à titre d'exemple, la composition selon l'invention peut contenir des filtres UV-B liposolubles, susceptibles de contribuer à la stabilisation ou à la solubilisation des filtres à large spectre ou encore de se stabiliser réciproquement et par ce fait, augmenter le facteur de protection solaire (FPS ou « SPF » en anglais).

Avantageusement, de tels filtres correspondent aux désignations INCI suivantes: homosalate, octocrylene, ethylhexyl salicylate, ethylhexyl triazone.

Dans un mode de réalisation préféré, la composition selon l'invention comprend de l'ethylhexyl triazone.

Dans un autre mode de réalisation, le filtre UV-B liposoluble est l'α-(triméthylsilyl)-ω-(triméthylsilyloxy)poly[oxy(diméthyl)silylène]-co-[oxy(méthyl)(2-{4-[2,2-bis(éthoxycarbonyl)vinyl]phénoxy}-1-méthylèneéthyl)silylène]-co-[oxy(méthyl)(2-(4-[2,2-bis(éthoxycarbonyl)vinyl]phénoxy)prop-1-ényl)silylène], un polymère siliconé capable de filtrer dans l'UV-B (INCI : polysilicone-15).

Dans un mode de réalisation préféré, la composition selon l'invention comprend au moins un filtre UV-B choisi dans le groupe suivant de composés identifiés par leur désignation INCI : homosalate, ethylhexyl salicylate, ethylhexyl triazone, polysilicone-15, ou leurs mélanges.

Dans un mode de réalisation particulier, la composition est exempte des filtres suivants : 4-methylbenzylidene camphor, benzophenone-2, benzophenone-3, ethylhexyl methoxycinnamate, octocrylene.

Dans un mode de réalisation avantageux, la composition selon l'invention comprend au moins un filtre UV-A, afin d'assurer une filtration complète de la partie nuisible du spectre solaire.

Des filtres UV-A avantageux au sens de la présente invention sont le butyl methoxydibenzoylmethane (INCI) et le diethylamino hydroxybenzoyl hexyl benzoate (INCI).

Dans un mode de réalisation particulier, le filtre UV-A est le bis-(diethylaminohydroxybenzoyl benzoyl) piperazine (INCI) (numéro CAS 919803-06-8).

Ainsi et dans un mode de réalisation préféré, la composition selon l'invention comprend au moins un filtre choisi dans le groupe suivant de composés identifiés par leur désignation inci : butyl methoxydibenzoylmethane, diethylamino hydroxybenzoyl hexyl benzoate, bis-(diethylaminohydroxybenzoyl benzoyl) piperazine, ou leurs mélanges.

D'autres filtres UV avantageux au sens de la présente invention sont des filtres hydrosolubles, comme par exemple :
- le filtre correspondant à la désignation INCI disodium phenyl dibenzimidazole tetrasulfonate.
- le filtre correspondant à la désignation INCI phenylbenzimidazole sulfonic acid,
Dans un mode de réalisation préféré, la composition selon l'invention comprend au moins un filtre hydrosoluble choisi dans le groupe suivant de composés identifiés par leur désignation INCI : disodium phenyl dibenzimidazole tetrasulfonate, phenylbenzimidazole sulfonic acid, ou leurs mélanges.

Avantageusement, les filtres minéraux inorganiques, ou écrans minéraux, sont des oxydes métalliques et/ou d'autres composés difficilement solubles ou insolubles dans l'eau, en particulier les oxydes de titane (TiO₂), de zinc (ZnO), de fer (Fe₂O₃), de zirconium (ZrO₂), de silicium (SiO₂), de manganèse (par exemple MnO), d'aluminium (Al₂O₃), ou de cérium (Ce₂O₃).

Selon un mode de réalisation particulier, les filtres minéraux inorganiques peuvent être utilisés sous forme de prédispersion huileuse ou aqueuse disponible sur le marché. Ces prédispersions peuvent être additionnées avantageusement d'auxiliaires de dispersion et/ou de médiateurs de solubilisation.

Les filtres minéraux inorganiques peuvent également être traités en surface ou encapsulés, afin de leur conférer un caractère hydrophile, amphiphile ou hydrophobe. Ce traitement de surface peut consister en ce que les filtres minéraux soient dotés d'une mince pellicule inorganique et/ou organique hydrophile et/ou hydrophobe.

Dans un mode de réalisation préféré, la composition selon l'invention comprend au moins un écran minéral choisi dans le groupe suivant de composés identifiés par leur désignation INCI : Zinc oxide, Titanium dioxide, ou leurs mélanges.

Les listes des filtres UV cités pouvant être mis en oeuvre au sens de la présente invention sont bien entendu donnés à titre indicatif et non limitatif.

De manière avantageuse, les filtres UV tels que décrits ci-dessus, présents dans la composition selon l'invention, représentent entre 0,1% et 30% en poids total de la composition, avantageusement entre 0,5 % et 20 %, encore plus avantageusement entre 1% et 15%.

Selon un mode de réalisation particulier, la composition selon l'invention présente un facteur de protection solaire (« SPF » ou FPS) supérieur ou égal à 10, de préférence supérieur ou égal à 20, avantageusement supérieur ou égal à 30, encore plus avantageusement supérieur ou égal à 50.

Selon un mode de réalisation préféré, la composition selon l'invention comporte un ratio de protection UV-A/UV-B égal ou supérieur à 1/3.

La composition solaire selon l'invention comprend au moins un solubilisants de filtres solaires.

La composition selon l'invention, peut en outre comprendre un « booster » de SPF, c'est-à-dire un agent amplificateur du facteur de protection solaire, et/ou un photostabilisant, c'est à dire un ingrédient qui permet d'augmenter le SPF ou de photostabiliser les filtres, un tel ingrédient n'étant pas considéré lui-même comme un filtre solaire. On peut par exemple citer :
- le butyloctyl salicylate (INCI), photostabilisant représentant avantageusement entre 0,01% et 10% en poids total de la composition, encore plus avantageusement entre 0,1% et 2%.;
- le benzotriazolyl dodecyl p-cresol (INCI), photostabilisant représentant avantageusement entre 0,01% et 10% en poids total de la composition, encore plus avantageusement entre 0,1% et 2%. - le pongamol (INCI), molécule végétale absorbant dans les UV-A, représentant avantageusement entre 0,5 et 2% en poids total de la composition, encore plus avantageusement de l'ordre de 1%.
- l'ethylhexyl methoxycrylene (INCI), photostabilisant, solubilisant et « booster » de SPF représentant avantageusement entre 1% et 5% en poids total de la composition.;
- un copolymère de styrène acrylate (INCI : styrene/acrylate copolymer), représentant de préférence entre 1% et 10% en poids total de la composition selon l'invention.
- le diethylhexyl syringylidene malonate (INCI), représentant avantageusement entre 1% et 10% en poids total de la composition. - un polyester hydrodispersible, correspondant aux désignations INCI polyester-5 (and) Sodium silicoaluminate, représentant avantageusement entre 1% et 10% en poids total de la composition,
- un copolymère d'acrylate ayant une température de transition vitreuse de -5°C à - 15°C telle que mesurée par calorimétrie différentielle à balayage, ledit copolymère représentant avantageusement entre 1% et 10% en poids total de la composition.

Selon un mode de réalisation particulier, la composition de l'invention comprend, en outre, une particule avantageusement colorée à effet flouteur ou un mélange de plusieurs particules à effet flouteur. Ces particules à effet flouteur sont également désignées « particules à effet soft-focus » ou encore « particules à effet de flou ».

Au sens de l'invention, par « particule à effet flouteur », on désigne une particule, avantageusement colorée, destinée à donner plus de transparence au teint et un effet de flou. En particulier, la particule à effet flouteur permet à la composition qui la contient d'atténuer, par effet optique, le microrelief cutané, et en particulier les défauts cutanés tels que les taches, les rides, les ridules.

Plusieurs particules à effet soft-focus ou flouteur sont disponibles sur les marchés. A titre d'exemple, on peut citer les matières premières de la gamme Ronasphere à base de substrat silice, commercialisée par la société MERCK, , ainsi que plusieurs matières premières commercialisées par les sociétés JGC-C&C (gamme Coverleaf à base de substrat séricite ou séricite/talc), NIHON KOKEN (gamme Relief Color, Silséem à base de substrat mica/silice), ou MIYOSHI KASEI (gamme PC-Bail à base de substrat silice).

Selon encore un autre mode de réalisation, la matière première KSP-100 commercialisée par la société SHINETSU correspondant à la désignation INCI Vinyl dimethicone/methicone silsesquioxane crosspolymer peut être utilisée en qualité de particule à effet flouteur.

De préférence, les particules à effet flouteur comprises dans la composition de l'invention représentent entre 0,5% et 20% en poids total de la composition, avantageusement entre 1% et 10%, encore plus avantageusement entre 1% et 5%

Selon un mode de réalisation particulier, la composition selon l'invention peut comprendre, en outre, tout corps gras usuellement utilisé dans le domaine cosmétique. On peut notamment citer les corps gras siliconés tels que les huiles, les gommes et les cires de silicone ; ainsi que les corps gras non siliconés tels que les huiles et les cires d'origine végétale, minérale, animale et/ou synthétique. Les huiles peuvent être volatiles ou non volatiles. On peut encore citer les hydrocarbures, les esters et les éthers de synthèse, les alcools gras et les acides gras.

Selon un mode de réalisation particulier, la composition peut comprendre, en outre, un milieu aqueux, un milieu hydroalcoolique contenant un alcool tel que l'éthanol, ou un milieu organique comprenant des solvants organiques usuels tels que des alcools en C1-6, notamment l'éthanol et l'isopropanol, des glycols tels que le propylène glycol, des cétones.

Selon un mode de réalisation particulier, la composition selon l'invention comprend, en outre, au moins un émulsifiant classique, choisi parmi les émulsifiants amphotères, anioniques, cationiques ou non ioniques, utilisés seuls ou en mélange.

Selon un mode de réalisation particulier, la composition selon l'invention comprend, en outre, les adjuvants habituels dans le domaine considéré, tels que les épaississants ou gélifiants hydrophiles ou lipophiles, les additifs hydrophiles ou lipophiles, les actifs notamment cosmétiques, les conservateurs, les antioxydants, les parfums, les charges, les pigments, , les absorbeurs d'odeur, les colorants, les hydratants (glycérine), des vitamines, des acides gras essentiels, des polymères liposolubles notamment hydrocarbonés, les opacifiants, les stabilisants, les séquestrants, les conditionneurs et les agents propulseurs.

Bien entendu, l'homme du métier veillera à choisir ce ou ces éventuels adjuvants ou excipients complémentaires, et/ou leur quantité, de manière telle que les propriétés avantageuses de la composition selon l'invention ne soient pas, ou substantiellement pas, altérées par l'adjonction envisagée.

La composition selon l'invention est avantageusement formulée pour être cosmétiquement acceptable, c'est-à-dire compatible avec la peau, les muqueuses, les cheveux et le cuir chevelu. De préférence, la composition de l'invention est une composition pour application cutanée ou à usage topique.

Par « composition pour application cutanée » ou « à usage topique » on désigne une composition compatible avec une application sur la peau, les muqueuses, les cheveux et/ou le cuir chevelu, de préférence la peau humaine.

La composition de l'invention peut se présenter sous toutes les formes galéniques appropriées pour une application topique, notamment sous forme de solution aqueuse, hydroalcoolique, organique ou huileuse ; de suspension ou de dispersion dans des solvants ou des corps gras, de type lotion ou sérum ; sous forme de dispersion vésiculaire ; sous forme d'émulsion eau dans huile (E/H), huile dans eau (H/E) ou multiple telle qu'une émulsion eau dans huile dans eau (E/H/E). L'émulsion peut être plus au moins épaisse et se présente sous forme de crème ou de lait ; la composition de l'invention peut se présenter également sous forme de pommade, de gel, de bâtonnet solide, de produits anhydres pâteux ou solides, de mousse, notamment aérosol, de composition biphasique ou encore de composition pulvérisable.

La forme galénique de la composition et son mode de préparation, et par conséquent les excipients appropriés à la composition de l'invention, peuvent être choisis par l'Homme du métier sur la base de ses connaissances générales en fonction du type de composition recherchée.

Il peut être particulièrement avantageux de formuler la composition de l'invention de façon qu'elle soit pulvérisable. Ceci peut être réalisé par exemple par la formulation d'émulsions spécifiques comprenant des combinaisons particulières d'excipients. Selon un autre aspect, l'invention concerne un procédé de traitement cosmétique consistant à appliquer sur la peau une composition telle que définie ci-dessus. Selon un autre aspect, l'invention concerne une composition, avantageusement cosmétique, comprenant au moins un glycosylcéramide, avantageusement un monoglycosylcéramide et/ou un polyglycosylcéramide, pour la prévention et/ou le traitement de la dermatite atopique.

Selon un mode de réalisation particulier, le au moins un un glycosylcéramide, avantageusement un monoglycosylcéramide et/ou un polyglycosylcéramide, est tel que décrit précédemment.

L'invention concerne également la composition telle que définie précédemment pour l'utilisation dans une méthode de traitement de la dermatite atopique par une application sur la peau.

Selon un autre aspect, l'invention concerne une composition, avantageusement cosmétique, comprenant :
- au moins un acide aminé basique sélectionné dans le groupe constitué de arginine, lysine, histidine, ornithine, hydroxyproline et un de leurs sels et
- au moins un glycosylcéramide,
pour utilisation pour la prévention et/ou le traitement de la dermatite atopique.

Selon un autre aspect particulier, l'invention concerne une composition comprenant au moins un glycosylcéramide, avantageusement un monoglycosylcéramide et/ou un polyglycosylcéramide, de préférence en association avec au moins un acide aminé basique sélectionné dans le groupe constitué de arginine, lysine, histidine, ornithine, hydroxyproline et un de leurs sels, pour utilisation pour :
- induire l'expression, ou augmenter l'expression, d'au moins un gène codant une protéine transmembranaire constitutive des jonctions serrées, avantageusement la claudine-1 ; et/ou
- induire une augmentation de la teneur en au moins une protéine transmembranaire constitutive des jonctions serrées, avantageusement la claudine-1 ; et/ou
- améliorer l'intégrité des jonctions serrées ; et/ou
- renforcer, améliorer, la fonction barrière de la peau.

Selon un mode de réalisation particulier, l'acide aminé basique compris dans la composition pour son utilisation selon l'invention est l'arginine ou un de ses sels.

Avantageusement, la composition pour son utilisation selon l'invention contient de la L-arginine.

Selon un mode de réalisation particulier, la composition pour son utilisation selon l'invention comprend au moins un glycosylcéramide correspondant à un monoglycosylcéramide et/ou polyglycosylcéramide, avantageusement un monoglycosylcéramide et/ou tétraglycosylcéramide.

Selon un mode de réalisation préféré, la composition pour son utilisation selon l'invention comprend des glycosylcéramides sous forme monoglycosylcéramide et tétraglycosylcéramide.

Selon un mode de réalisation particulier, la composition pour son utilisation selon l'invention comprend un monoglycosylcéramide correspondant à un acide glucuronique (ou acide gluconique) lié de façon covalente par une liaison β-osidique à un céramide.

Selon un mode de réalisation particulier, le polyglycosylcéramide compris dans la composition pour son utilisation selon l'invention correspond à un oligosaccharide lié de façon covalente par une liaison β-osidique à un céramide.

Avantageusement, le polyglycosylcéramide compris dans la composition pour son utilisation selon l'invention est un tétraglycosylcéramide dont l'oligosaccharide correspond à l'enchainement des oses suivant : acide glucuronique-glucosamine-galactose-mannose (ou GlcA-GlcN-Gal-Man).

Le ou les glycosylcéramides compris dans la composition pour son utilisation selon l'invention peuvent être d'origine végétale, chimique, ou bien obtenus par biotechnologie.

Avantageusement, la composition pour son utilisation selon l'invention comprend au moins un glycosylcéramide d'origine biotechnologique.

Avantageusement, tous les glycosylcéramides compris dans la composition pour son utilisation selon l'invention sont d'origine biotechnologique.

Selon un mode de réalisation particulier, le au moins un acide aminé basique, avantageusement l'arginine ou un de ses sels, représente entre 0,001% et 2% en poids total de la composition, avantageusement entre 0,01% et 0,5%.

Selon un autre mode de réalisation particulier, le au moins un glycosylcéramide, avantageusement des glycosylcéramides sous forme monoglycosylcéramide et tétraglycosylcéramide, représente entre 0,0001% et 1% en poids total de la composition, avantageusement entre 0,001% et 0,1%.

Selon un mode de réalisation particulier, la composition pour son utilisation selon l'invention est celle décrite précédemment (voir le premier aspect de l'invention).

Avantageusement et en relation avec ces différentes applications thérapeutiques, la composition de l'invention est sous une forme galénique adaptée à une administration par voie topique.

Une manière de mettre en évidence l'effet de la composition selon l'invention sur la prévention et/ou le traitement de la dermatite atopique est, par exemple, une évaluation de l'expression d'au moins un gène codant pour une protéine transmembranaire constitutive des jonctions serrées comme la claudine-1. Alternativement, il peut s'agir d'une analyse qualitative et/ou quantitative visant au moins une protéine transmembranaire constitutive des jonctions serrées comme la claudine-1. A titre d'exemple, on peut citer une analyse par chromatographie et/ou spectrométrie de masse, par western blot ou encore par marquage immunohistochimique.

La manière dont l'invention peut être réalisée et les avantages qui en découlent ressortiront mieux des exemples de réalisation qui suivent, donnés à titre indicatif et non limitatif, à l'appui de la figure annexée.

### Description des figures

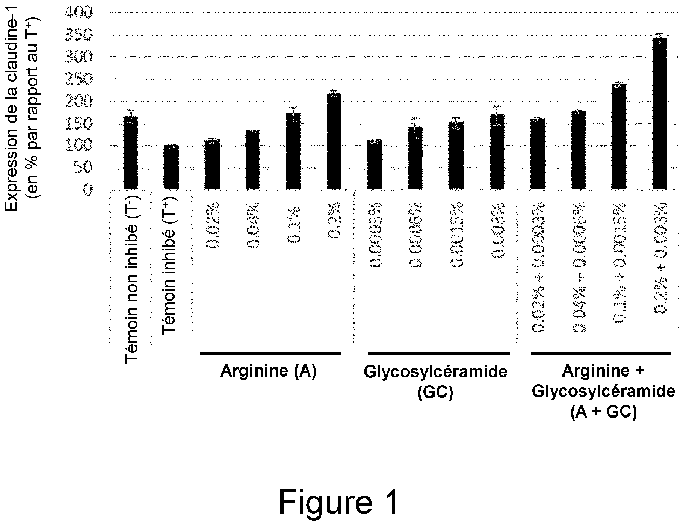

La figure 1 montre l'effet de l'arginine (A), des glycosylcéramides (GC), et de leurs associations (A + GC) sur l'expression de la claudine-1 dans un modèle cellulaire de dermatite atopique. Les conditions « témoin » correspondent au témoin négatif (non inhibé ou T⁻) et au témoin positif (inhibé ou T⁺). Le pourcentage d'induction de la synthèse de la claudine-1 est rapporté au témoin positif inhibé (100%). ** indique un seuil de significativité statistique p compris entre 0,001 et 0,01 (très significatif) et *** indique un seuil de significativité statistique p < 0,001 (extrêmement significatif).

### Exemples de réalisation

### EXEMPLE 1 : COMPOSITION COSMETIQUE AU SENS DE L'INVENTION - SPRAY

Une composition selon l'invention est décrite dans le tableau 1.

| **Ingrédient ( INCI)** | **Poids (en %)** |
|---|---|
| Aqua/water/eau | qsp100 |
| Glycerin | 15,00 |
| Dipropylene glycol | 9,00 |
| Isostearyl isostearate | 2,00 |
| Propylheptyl caprylate | 1,999 |
| Squalane | 1,00 |
| Sodium cocoyl glutamate | 0,8825 |
| Glycyrrhetinic acid | 0,50 |
| Pentylene glycol | 0,50 |
| Ornithine | 0,30 |
| Disodium EDTA | 0,20 |
| Citric acid | 0,17 |
| Propanediol | 0,125 |
| Xanthan gum | 0,10 |
| Tocopherol | 0,036 |
| Lecithin | 0,0201 |
| *Helianthus annuus* (sunflower) seed oil | 0,015 |
| Glycosphingolipids | 0,007 |

### EXEMPLE 2 : COMPOSITION COSMETIQUE AU SENS DE L'INVENTION - GEL

Une composition selon l'invention est décrite dans le tableau 2.

| **Ingrédient ( INCI)** | **Poids (en %)** |
|---|---|
| Aqua/Water/Eau | qsp 100 |
| Glycerin | 2,00 |
| Hydroxyethyl Acrylate/Sodium Acryloyldimethyl Taurate Copolymer | 1,125 |
| Isohexadecane | 0,765 |
| Titanium Dioxide [Nano] | 0,375 |
| Phenoxyethanol | 0,35 |
| 1,2-hexanediol | 0,26 |
| Lysine | 0,20 |
| Polysorbate 20 | 0,165 |
| Alumina | 0,045 |
| Glycosphingolipids | 0,005 |

### EXEMPLE 3 : COMPOSITION COSMETIQUE AU SENS DE L'INVENTION - EMULSION H/E

Une composition selon l'invention est décrite dans le tableau 3.

| **Ingredient ( INCI)** | **Poids (en %)** |
|---|---|
| Aqua/water/eau | qsp 100 |
| Glycerin | 13,00 |
| *Brassica campestris* (rapeseed) seed oil | 11,00 |
| Paraffinum liquidum/mineral oil/huile minerale | 8,00 |
| Behenyl alcohol | 2,00 |
| Dipropylene glycol | 2,00 |
| Sucrose stéarate | 2,00 |
| *Simmondsia chinensis* (jojoba) seed oil | 1,00 |
| Hydroxyethyl acrylate/sodium acryloyldimethyl taurate copolymer | 0,616 |
| Pentylene glycol | 0,50 |
| Acrylates/c10-30 alkyl acrylate crosspolymer | 0,30 |
| Caprylyl glycol | 0,2545 |
| 1,2-hexanediol | 0,25 |
| **Arginine** | **0,20** |
| Sodium citrate | 0,20 |
| Dipotassium glycyrrhizate | 0,10 |
| Glycyrrhetinic acid | 0,10 |
| Sodium lauroyl lactylate | 0,10 |
| Polysorbate 60 | 0,042 |
| Sorbitan isostearate | 0,042 |
| Citric acid | 0,040 |
| Tocopherol | 0,035 |
| Methylpropanediol | 0,0246 |
| Phytosphingosine | **0,02** |
| Triethyl citrate | 0,02 |
| *Helianthus annuus* (sunflower) seed oil | 0,015 |
| Ceramide NP | 0,01 |
| Ceramide AP | **0,006** |
| Cholestérol | 0,005 |
| Carbomer | 0,004 |
| Xanthan gum | 0,004 |
| **Glycosphingolipids** | **0,003** |
| Phenylpropanol | 0,0009 |
| Ceramide EOP | **0,00001** |

### EXEMPLE 4 : EFFET DE LA COMPOSITION SELON L'INVENTION SUR L'EXPRESSION DE LA PROTEINE CLAUDINE-1 DANS UN MODELE CELLULAIRE IN VITRO DE DERMATITE ATOPIQUE

### 1. Matériel et méthodes

### 1. 1. Modèle biologique et lignées cellulaires

### Lignée cellulaire :

- type cellulaire : Kératinocytes épidermiques humains normaux (NHEK ; référence : Bioalternatives K34 1), utilisés au 3ème passage- conditions de culture : 37°C, 5% CO₂
- milieu de culture :Kératinocyte-SFM complémenté avec le facteur de croissance épidermique *(Epidermal Growth Factor* ou EGF) à une concentration de 0,25 ng/ml, un extrait Pituitaire (EP) à une concentration de 25 µg/ml et de la gentamycine à une concentration de 25 µg/ml
- milieu d'essai mis en oeuvre pour cet exemple :Kératinocyte -SFM complémenté avec de la gentamycine à une concentration de 25 µg/ml.

Une culture de kératinocytes humains normaux est réalisée afin de tester les substances ; brièvement le protocole est le suivant :
- induction de la différenciation des kératinocytes par le calcium à 0,75 mM pendant 24h ;
- traitement avec la composés du, 1 heure avant l'induction par les alarmines ;
- traitement par les alarmines suivantes : histamine, IL-33 et IL-1β, dans les concentrations respectives suivantes : 50 µM, 100 ng/ml et 100 ng/ml, afin d'induire le phénotype de dermatite atopique, caractérisé par une baisse de l'expression de la claudine-1.

Un immunomarquage de la claudine-1 est ensuite réalisé.

### 1.2. Composés actifs de la composition selon l'invention

Les composés mis en oeuvre, seuls ou en combinaison, dans cet exemple sont représentés dans le tableau 4 ci-dessous.

| **Composés actifs selon l'invention** | **Solution stock** | **Concentrations testées** |
|---|---|---|
| Arginine | 10% en eau ultrapure | 0,02%, 0,04%, 0,1% et 0,2% |
| Glycosylcéramides | 5% en eau ultrapure | 0,0003%, 0,0006%, 0,0015% et 0,003% |

### 1.3. Test de cytotoxicité

Des tests préliminaires de cytotoxicité sont réalisés sur des kératinocytes humains normaux en milieu d'essai comprenant en outre du CaCl₂ à une concentration de 0,75 mM. Ces tests préliminaires couplent la méthode de réduction du sel de tétrazolium MTT (bromure de 3-(4,5-dimethylthiazol-2-yl)-2,5-diphenyl tetrazolium) et des observations morphologiques au microscope.

A la fin du traitement (72 h d'incubation), les cellules ont été incubées en présence de MTT dont la transformation en cristaux bleus de formazan est proportionnelle à l'activité de la succinate déshydrogénase (enzyme mitochondriale). Apres dissociation des cellules et solubilisation du formazan par ajout de DMSO, la densité optique (DO), représentative du nombre de cellules vivantes et de leur activité métabolique, a été mesurée avec un spectrophotomètre (VERSAmax, Molecular Devices) à 540 nm.

Les résultats du test de viabilité au MTT et l'observation des tapis cellulaires ont conduit à sélectionner les concentrations des composés actifs selon la composition de l'invention à tester dans la suite de l'étude pour le composé d'intérêt (voir tableau 2).

### 1.4. Culture et traitement

Les kératinocytes ont été ensemencés en plaques 96 puits et cultivés en milieu de culture pendant 72 heures, puis en milieu d'essai contenant l'inducteur de différenciation CaCl₂ (0,75 mM) pendant 24 heures supplémentaires. Le milieu a ensuite été remplacé par du milieu d'essai contenant l'inducteur CaCl₂ (0,75 mM) et contenant ou non (témoin) les composés actifs de l'invention, seuls ou en combinaison, ou le contrôle solvant (DMSO testé à 0,02%) puis les cellules ont été pré- incubées pendant 1 heure. Le mélange des alarmines : histamine, IL-33 et IL-1β (dans les concentrations respectives suivantes : 50 µM, 100 ng/ml et 100 ng/ml) a ensuite été ajouté et les cellules ont été incubées pendant 72 heures. En parallèle, une condition témoin non stimulée/différenciée et non inhibée (milieu d'essai sans CaCl₂ et sans alarmines) et une condition témoin non inhibée (milieu d'essai avec CaCl₂ et sans alarmines) ont été réalisées.

Pour chaque condition, l'analyse a été réalisée à partir de 30 quantifications d'images (n=30), excepté pour les conditions témoin réalisées à partir de 60 quantifications d'images (n=60). Une première exploitation des résultats a été réalisée en prenant en compte l'ensemble des quantifications puis une seconde uniquement avec les résultats par réplicas de culture soit en triplicates (n =3), excepté pour les conditions témoin réalisées en hexoplicates (n=6).

### 1.5. Immunomarguage in situ

Apres incubation, le milieu a été éliminé et les cellules ont été rincées, fixées et perméabilisées. Les cellules ont ensuite été marquées avec l'anticorps primaire dirigé contre la claudine-1. Cet anticorps a ensuite été révélé par un anticorps secondaire couplé à un fluorochrome (GAM-Alexa 488). En parallèle, les noyaux des cellules ont été colorés par le Hoechst 33258 (bis-benzimide).

### 1.6. Observations au microscope et analyses d'images

L'acquisition des images a été réalisée avec un système d'imagerie à haute résolution, INCell Analyzer^{™}2200 (GE Healthcare ; objectif x20). Pour chaque puits, 10 saisies d'images numérisées ont été effectuées.

Les marquages ont été quantifiés par mesure de l'intensité de fluorescence des protéines rapportée au nombre total de cellules identifiées par la coloration avec du bis-benzimide (intégration des données numériques par le logiciel Developer Toolbox 1.5, GE Health care).

### 1.7. Traitement des données

Les données brutes ont été transférées et traitées sous le logiciel Microsoft Excel^{®}.

Les comparaisons intergroupes ont été réalisées à l'aide du test-t de Student bilatéral non apparié. Les analyses statistiques peuvent être interprétées si n≥5. Cependant, pour n<5, les données calculées ne sont fournies qu'à titre indicatif.

Formules utilisées dans ce rapport :
- L'erreur standard de la moyenne (esm) représente l'écart de la moyenne de l'échantillon par rapport à la moyenne de la vraie population. L'esm est calculé en divisant l'écart-type (Sd) par la racine carrée de la taille de l'échantillon selon la formule suivante : esm = écart-type (Sd)/ √n ;
- Le pourcentage de viabilité est calculé selon la formule suivante : viabilité (%) = (DO compose / DO témoin) x 100 ;
- Le seuil de significativité statistique est le suivant : ns : > 0.0,5 (non significatif) ; * = 0,01 à 0,05 (significatif) ; ** = 0,001 à 0,01 (très significatif) ; *** = < 0,001. (extrêmement significatif). La significativité a été calculé en comparant statistiquement la différence entre résultats de actifs seuls et l'association, à chaque dose.

Les résultats sont représentés dans la figure 1.

### 2. Résultats

Dans les conditions témoins non stimulées/non différenciées par le calcium (CaCl₂), l'immunomarquage de la claudine-1 est présent dans quelques cellules de façon punctiforme dans le cytoplasme et parfois dans les membranes.

Comme attendu, la différenciation par le CaCl₂ à une concentration de 0,75 mM a induit une augmentation de l'expression de la protéine de jonctions serrées analysée (claudine-1). Cette induction est forte et nettement significative, et se traduit par une augmentation importante de l'expression de la claudine-1 au niveau membranaire mais également cytoplasmique.

Le traitement par le mélange d'alarmines (histamine + IL-1 + IL-33 testées à 50 µM + 100 ng/ml + 100 ng/ml) sur les kératinocytes stimulés/différenciés par le calcium (CaCl₂) à 0,75 mM, a induit un effet inhibiteur significatif de l'expression de la claudine-1 (moyenne d'environ 61,5% du témoin).

L'effet du traitement avec les composés actifs de l'invention, seuls ou en combinaison, sur l'expression de la claudine-1 sont représentés par la figure 1.

Il ressort de ces résultats que l'arginine (A) et les glycosylcéramides (GC) testés seuls (% par rapport au T⁺) inversent de façon significative l'effet inhibiteur du mélange d'alarmines sur l'expression de la claudine-1.

L'association d'arginine et de glycosylcéramides (A + GC) exerce un effet d'induction de l'expression de la claudine-1 plus importante que lorsque ces composés sont testés seuls. A toutes les combinaisons de concentration testées, l'augmentation était soit très significative (***) soit extrêmement significative (**) et, dans chaque cas plus que complémentaire, signifiant une synergie potentialisatrice inattendue de l'association de l'arginine et de glycosylcéramides dans l'induction de l'expression de la claudine-1 et, par conséquent, sur le renforcement de la fonction barrière de la peau et la prévention et/ou le traitement de la dermatite atopique.

### BIBLIOGRAPHIE

Batista, D.I.S., Perez, L., Orfali, R.L., Zaniboni, M.C., Samorano, L.P., Pereira, N.V., Sotto, M.N., Ishizaki, A.S., Oliveira, L.M.S., Sato, M.N., et al. (2015). Profile of skin barrier proteins (filaggrin, claudins 1 and 4) and Th1/Th2/Th17 cytokines in adults with atopic dermatitis. J. Eur. Acad. Dermatol. Venereol. 29: 1091-1095.

Brandner, J.M., Kief, S., Grund, C., Rendl, M., Houdek, P., Kuhn, C., Tschachler, E., Franke, W.W., and Moll, I. (2002). Organization and formation of the tight junction system in human epidermis and cultured keratinocytes. Eur. J. Cell Biol. 81: 253-263.

Cipriani, F., Dondi, A., and Ricci, G. (2014). Recent advances in epidemiology and prévention of atopic eczema. Off. Publ. Eur. Soc. Pediatr. Allergy Immunol. 25: 630-638.

Gruber, R., Börnchen, C., Rose, K., Daubmann, A., Volksdorf, T., Wladykowski, E., Vidal-Y-Sy, S., Peters, E.M., Danso, M., Bouwstra, J.A., et al. (2015). Diverse régulation of claudin-1 and claudin-4 in atopic dermatitis. Am. J. Pathol. 185: 2777-2789.

Halling-Overgaard, A.-S., Kezic, S., Jakasa, I., Engebretsen, K.A., Maibach, H., and Thyssen, J.P. (2017). Skin absorption through atopic dermatitis skin: a systematic review. Br. J. Dermatol. 177: 84-106.

Mack, M.R., and Kim, B.S. (2018). The Itch-Scratch Cycle: A Neuroimmune Perspective. Trends Immunol. 39: 980-991.

Mochizuki, H., Lavery, M.J., Nattkemper, L.A., Albornoz, C., Valdes Rodriguez, R., Stull, C., Weaver, L., Hamsher, J., Sanders, K.M., Chan, Y.H., et al. (2019). Impact of acute stress on itch sensation and scratching behaviour in patients with atopic dermatitis and healthy controls. Br. J. Dermatol. 180: 821-827.

Weidinger, S., Beck, L.A., Bieber, T., Kabashima, K., and Irvine, A.D. (2018). Atopic dermatitis. Nat. Rev. Dis. Primer 4: 1.

## Revendications

1. Composition comprenant :
- au moins un acide aminé basique sélectionné dans le groupe constitué de l'arginine, la lysine, l'histidine, l'ornithine, l'hydroxyproline et l'un de leurs sels ; et
- au moins un glycosylcéramide se présentant sous la forme d'un monoglycosylcéramide correspondant à un acide glucuronique lié de façon covalente à un céramide et/ou d'un polyglycosylcéramide correspondant à un oligosaccharide lié de façon covalente à un céramide.

2. Composition selon la revendication 1, **caractérisée en ce que** l'acide aminé basique est l'arginine ou l'un de ses sels.

3. Composition selon l'une quelconque des revendications précédentes, **caractérisée en ce que** le polyglycosylcéramide se présente sous la forme d'un tétraglycosylcéramide.

4. Composition selon l'une quelconque des revendications 1 ou 2, **caractérisée en ce que** le glycosylcéramide se présente sous la forme d'un monoglycosylcéramide et d'un tétraglycosylcéramide.

5. Composition selon la revendication 4, **caractérisée en ce que** :
- l'oligosaccharide lié de façon covalente au céramide est l'acide glucuronique-glucosam ine-galactose-mannose.

6. Composition selon l'une quelconque des revendications précédentes, **caractérisée en ce que** le au moins un glycosylcéramide est obtenu par extraction à partir de membranes d'au moins une espèce bactérienne.

7. Composition selon l'une quelconque des revendications précédentes, **caractérisée en ce que** le au moins un acide aminé basique, avantageusement l'arginine, représente entre 0,001% et 2% en poids total de la composition, avantageusement entre 0,01% et 0,5%.

8. Composition selon l'une quelconque des revendications précédentes, **caractérisée en ce que** le au moins un glycosylcéramide, représente entre 0,0001% et 1 % en poids total de la composition, avantageusement entre 0,001% et 0,1 %.

9. Composition selon l'une quelconque des revendications précédentes, **caractérisée en ce qu'**elle comprend, en outre :
- au moins un saponoside triterpénique ou l'un de ses dérivés choisi dans le groupe constitué de l'acide glycyrrhétinique, la glycyrrhizine, le stéaryl glycyrrhétinate et le dipotassium diglycyrrizinate ; et/ou
- au moins un tensioactif non-ionique sélectionné parmi les esters de sorbitan et/ou les esters de saccharose.

10. Composition selon la revendication 9, **caractérisée en ce que** :
- le au moins un saponoside triterpénique ou l'un de ses dérivés représente entre 0,0001% et 10% en poids total de la composition, avantageusement entre 0,01% et 5%, de préférence entre 0,05% et 1 % ; et/ou
- le au moins un tensioactif non-ionique représente 0,1% et 5% en poids total de la composition, avantageusement entre 1% et 3%.

11. Composition selon l'une quelconque des revendications 9 à 10, **caractérisée en ce que** le tensioactif non-ionique est le polysorbate 20 ou un sucrostéarate ayant une valeur de Balance Hydrophile Lipophile (HLB) d'au moins 16.

12. Composition selon l'une quelconque des revendications précédentes, **caractérisée en ce qu'**elle comprend, en outre, au moins un lipide apte à restaurer la barrière cutanée, avantageusement sélectionné dans le groupe constitué de :
- au moins un lipide exogène de la peau, avantageusement une huile végétale ;
- un mélange de constituants naturellement présents dans la peau comprenant des céramides 1, 3, 6 ; du cholestérol ; des acides gras libres et de la phytosphingosine ; et
- le squalane.

13. Composition selon l'une quelconque des revendications précédentes, **caractérisée en ce qu'**il s'agit d'une composition cosmétique.

14. Composition selon l'une quelconque des revendications précédentes, **caractérisée en ce qu'**elle se présente sous une forme adaptée à une administration par voie topique.

15. Composition comprenant :
- au moins un acide aminé basique sélectionné dans le groupe constitué de l'arginine, la lysine, l'histidine, l'ornithine, l'hydroxyproline et l'un de leurs sels ; et
- au moins un glycosylcéramide se présentant sous la forme d'un monoglycosylcéramide correspondant à un acide glucuronique lié de façon covalente à un céramide et/ou d'un polyglycosylcéramide correspondant à un oligosaccharide lié de façon covalente à un céramide.
pour la prévention et/ou le traitement de la dermatite atopique.

16. Composition selon l'une quelconque des revendications 1 à 14, pour utilisation pour la prévention et/ou le traitement de la dermatite atopique.

## Patentansprüche

1. Zusammensetzung, die Folgendes enthält:
- mindestens eine basische Aminosäure, die aus der Gruppe ausgewählt ist, die aus Arginin, Lysin, Histidin, Ornithin, Hydroxypyrolin und einem ihrer Salze besteht; und
- mindestens ein Glycosylceramid in Form eines Monoglycosylceramids, das einer Glucuronsäure entspricht, die kovalent an ein Ceramid gebunden ist, und/oder eines Polyclycosylceramids, das einem Oligosaccharid entspricht, das kovalent an ein Ceramid gebunden ist.

2. Zusammensetzung nach Anspruch 1, **dadurch gekennzeichnet, dass** die basische Aminosäure Arginin oder eines seiner Salze ist.

3. Zusammensetzung nach einem der vorhergehenden Ansprüche, **dadurch gekennzeichnet, dass** das Polyglycosylceramid in Form eines Tetraglycosylceramids vorliegt.

4. Zusammensetzung nach einem der Ansprüche 1 oder 2, **dadurch gekennzeichnet, dass** das Glycosylceramid in Form eines Monoglycosylceramids und eines Tetraglycosylceramids vorliegt.

5. Zusammensetzung nach Anspruch 4, **dadurch gekennzeichnet, dass**
- es sich bei dem kovalent an das Ceramid gebundene Oligosaccharid um Glucuronsäure-Glucosamin-Galactose-Mannose handelt.

6. Zusammensetzung nach einem der vorhergehenden Ansprüche, **dadurch gekennzeichnet, dass** das mindestens eine Glykosylceramid durch Extraktion aus den Membranen mindestens einer Bakterienart erhalten wird.

7. Zusammensetzung nach einem der vorhergehenden Ansprüche, **dadurch gekennzeichnet, dass** die mindestens eine basische Aminosäure, vorteilhafterweise Arginin, zwischen 0,001 % und 2 % des Gesamtgewichts der Zusammensetzung, vorteilhafterweise zwischen 0,01 % und 0,5 %, beträgt.

8. Zusammensetzung nach einem der vorhergehenden Ansprüche, **dadurch gekennzeichnet, dass** das mindestens eine Glykosylceramid zwischen 0,0001 % und 1 % des Gesamtgewichts der Zusammensetzung, vorteilhafterweise zwischen 0,001 % und 0,1 %, beträgt.

9. Zusammensetzung nach einem der vorhergehenden Ansprüche, **dadurch gekennzeichnet, dass** sie darüber hinaus Folgendes umfasst:
- mindestens ein Triterpen-Saponosid oder eines seiner Derivate, ausgewählt aus der Gruppe bestehend aus Glycyrrhetinsäure, Glycyrrhizin, Stearylglycyrrhetinat und Dikaliumdiglycyrrizinat; und/oder
- mindestens ein nicht-ionisches Tensid, ausgewählt aus Sorbitanestern und/oder Saccharoseestern.

10. Zusammensetzung nach Anspruch 9, **dadurch gekennzeichnet, dass**:
- das mindestens eine Triterpen-Saponosid oder eines seiner Derivate zwischen 0,0001 % und 10 % des Gesamtgewichts der Zusammensetzung beträgt, vorteilhafterweise zwischen 0,01 % und 5 %, vorzugsweise zwischen 0,05 % und 1 %; und/oder
- das mindestens eine nicht-ionische Tensid 0,1 % bis 5 % des Gesamtgewichts der Zusammensetzung beträgt, vorteilhafterweise zwischen 1 % und 3 %.

11. Zusammensetzung nach einem der Ansprüche 9 bis 10, **dadurch gekennzeichnet, dass** es sich bei dem nicht-ionischen Tensid um Polysorbat 20 oder um ein Sucrostearat mit einem Hydrophil-Lipophil-Gleichgewichtswert (HLB) von mindestens 16 handelt.

12. Zusammensetzung nach einem der vorhergehenden Ansprüche, **dadurch gekennzeichnet, dass** sie darüber hinaus mindestens ein Lipid enthält, das zur Wiederherstellung der Hautbarriere geeignet ist, vorteilhafterweise ausgewählt aus der Gruppe bestehend aus:
- mindestens einem exogenen Lipid der Haut, vorteilhafterweise ein Pflanzenöl;
- einem Gemisch aus natürlich in der Haut vorkommenden Bestandteilen, das Ceramide 1, 3, 6; Cholesterin; freie Fettsäuren und Phytosphingosin umfasst; und
- Squalan

13. Zusammensetzung nach einem der vorhergehenden Ansprüche, **dadurch gekennzeichnet, dass** es sich um eine kosmetische Zusammensetzung handelt.

14. Zusammensetzung nach einem der vorhergehenden Ansprüche, **dadurch gekennzeichnet, dass** sie in einer Form vorliegt, die für eine Verabreichung auf topischem Weg geeignet ist.

15. Zusammensetzung, die Folgendes umfasst:
- mindestens eine basische Aminosäure, die aus der Gruppe ausgewählt ist, die aus Arginin, Lysin, Histidin, Ornithin, Hydroxyprolin und einem ihrer Salze besteht; und
- mindestens ein Glykosylceramid in Form eines Monoglykosylceramids, das einer Glucuronsäure entspricht, die kovalent an ein Ceramid gebunden ist, und/oder in Form eines Polyglykosylceramids, das einem Oligosaccharid entspricht, das kovalent an ein Ceramid gebunden ist.

16. Zusammensetzung nach einem der Ansprüche 1 bis 14 zur Verwendung bei der Vorbeugung und/oder Behandlung von atopischer Dermatitis.

## Claims

1. Composition comprising:
- at least one basic amino acid selected from the group constituted by arginine, lysine, histidine, ornithine, hydroxyproline and one of their salts; and
- at least one glycosylceramide in the form of a monoglycosylceramide corresponding to a glucuronic acid covalently bonded to a ceramide and/or a polyglycosylceramide corresponding to an oligosaccharide covalently bonded to a ceramide.

2. Composition as claimed in claim 1, **characterized in that** the basic amino acid is arginine or one of its salts.

3. Composition as claimed in any one of the preceding claims, **characterized in that** the polyglycosylceramide is in the form of a tetraglycosylceramide.

4. Composition as claimed in claim 1 or claim 2, **characterized in that** the glycosylceramide is in the form of a monoglycosylceramide and a tetraglycosylceramide.

5. Composition as claimed in claim 4, **characterized in that**:
- the oligosaccharide which is covalently bonded to the ceramide is glucuronic acid-glucosamine-galactose-mannose.

6. Composition as claimed in any one of the preceding claims, **characterized in that** the at least one glycosylceramide is obtained by extraction from membranes of at least one bacterial species.

7. Composition as claimed in any one of the preceding claims, **characterized in that** the at least one basic amino acid, advantageously arginine, represents between 0.001% and 2% of the total weight of the composition, advantageously between 0.01% and 0.5%.

8. Composition as claimed in any one of the preceding claims, **characterized in that** the at least one glycosylceramide represents between 0.0001% and 1% of the total weight of the composition, advantageously between 0.001% and 0.1%.

9. Composition as claimed in any one of the preceding claims, **characterized in that** it comprises, in addition:
- at least one triterpene saponoside or one of its derivatives selected from the group constituted by glycyrrhetinic acid, glycyrrhizin, stearyl glycyrrhetinate and dipotassium diglycyrrizinate; and/or
- at least one non-ionic surfactant selected from sorbitan esters and/or saccharose esters.

10. Composition as claimed in claim 9, **characterized in that**:
- the at least one triterpene saponoside or one of its derivatives represents between 0.0001% and 10% of the total weight of the composition, advantageously between 0.01% and 5%, preferably between 0.05% and 1%; and/or
- the at least one non-ionic surfactant represents between 0.1% and 5% of the total weight of the composition, advantageously between 1% and 3%.

11. Composition as claimed in claim 9 or claim 10, **characterized in that** the non-ionic surfactant is polysorbate 20 or a sucrostearate with a Hydrophilic Lipophilic Balance (HLB) of at least 16.

12. Composition as claimed in any one of the preceding claims, **characterized in that** it additionally comprises at least one lipid which is capable of restoring the skin barrier, advantageously selected from the group constituted by:
- at least one lipid which is exogenous to the skin, advantageously a vegetable oil;
- a mixture of constituents which are naturally present in the skin, comprising ceramide-1, ceramide-3, ceramide-6; cholesterol; free fatty acids and phytosphingosine; and
- squalane.

13. Composition as claimed in any one of the preceding claims, **characterized in that** it is a cosmetic composition.

14. Composition as claimed in any one of the preceding claims, **characterized in that** it is in a form which is suitable for topical administration.

15. Composition comprising
- at least one basic amino acid selected from the group constituted by arginine, lysine, histidine, ornithine, hydroxyproline and one of their salts; and
- at least one glycosylceramide in the form of a monoglycosylceramide corresponding to a glucuronic acid covalently bonded to a ceramide and/or a polyglycosylceramide corresponding to an oligosaccharide covalently bonded to a ceramide for the prevention and/or treatment of atopic dermatitis.

16. Composition as claimed in any one of claims 1 to 14, for use in the prevention and/or treatment of atopic dermatitis.
